# FASCICULE DE BREVET EUROPEEN

(11) **EP 4 366 670 B1**
(45) Date de publication et mention de la délivrance du brevet: **23.07.2025**
(21) Numéro de dépôt: 22747659.5
(22) Date de dépôt: 07.07.2022
(51) Int. Cl.: A61F 5/01, A61F 13/10, A44B 18/00

(54) **BANDE DE COMPRESSION AUTO-AGRIPPANTE**
SELBSTSICHERNDER KOMPRESSIONSSTREIFEN
SELF-FASTENING COMPRESSION STRIP

(30) Priorité: 09.07.2021 FR 2107467
(43) Date de publication de la demande: 15.05.2024
(73) Titulaire: Thuasne, 92300 Levallois Perret (FR)
(72) Inventeur: AGUD, Adrien, 07610 LEMPS (FR); BREUIL, Laurent, 42240 UNIEUX (FR)
(74) Mandataire: Lavoix
(86) Numéro de dépôt international: PCT/EP2022/068969
(87) Numéro de publication internationale: WO 2023/281005

(56) Documents cités:
- WO-A1-2015/133481
- GB-A- 1 300 077
- US-A- 5 480 709
- US-A1- 2017 065 449
- US-A1- 2018 073 169
- US-B1- 6 443 187

## Description

La présente invention concerne une bande de compression thérapeutique destinée à être enroulée sur au moins une partie d'un membre d'un patient.

L'invention concerne également un procédé de réalisation d'une telle bande de compression.

Il est connu d'appliquer une pression déterminée sur un membre ou sur une partie d'un membre d'un patient par exemple dans le cadre du traitement ou de la prévention de pathologies veineuses.

Pour appliquer la pression, des articles de compression, tels que des bas, collants, chaussettes, manchons de compression ou autre, sont généralement utilisés. De tels articles sont dimensionnés pour appliquer la pression souhaitée et un article particulier applique toujours une même pression de contention. Les articles sont ainsi proposés dans des tailles différentes adaptées pour appliquer différentes pressions de compression. Le choix d'un type d'article de compression et de la taille de celui-ci se fait en fonction de la partie de membre sur laquelle le traitement doit être appliqué et en fonction de la pression souhaitée. Le document US5480709, par exemple, décrit un tel type d'article de compression.

Cependant, l'utilisation de tels articles n'est pas toujours possible, par exemple lorsqu'un patient ne peut pas enfiler l'article de compression. Ceci est particulièrement le cas lorsqu'un patient souffre d'une entorse en phase aigüe, d'un ulcère veineux actif ou d'autres symptômes rendant l'enfilage ou le port d'un article de compression trop douloureux.

Dans un tel cas de figure, il est connu d'utiliser une ou plusieurs bandes de compression à la place d'un article de compression à enfiler. Une telle bande de compression est appliquée sur la partie du membre à traiter en étant enroulée autour de celle-ci en se recouvrant partiellement sur elle-même.

La pose et le maintien d'une telle bande de compression sont cependant compliqués car la bande doit être enroulée tout en étant étirée pour appliquer la pression souhaitée et cet étirement doit être maintenu alors que la bande est enroulée. Une fois que la bande est posée, il convient de la fixer sur le membre pour la maintenir en place et appliquer le traitement de compression. Pour ce faire, des agrafes d'attache sont par exemple utilisées pour fixer la bande de compression sur elle-même à une extrémité de celle-ci. Cependant, un tel dispositif de fixation n'est pas satisfaisant car il nécessite une pièce supplémentaire à fixer sur la bande de compression et que la fixation assurée n'est pas de nature à empêcher un glissement de la bande le long du membre, en particulier après que la bande a été portée un certain temps, ce qui réduit l'efficacité de la compression appliquée. Également, il est fréquent que ces agrafes blessent le patient ou les mains de la personne qui les positionne.

Pour pallier ces inconvénients, il a été proposé des bandes de compression « cohésive », c'est-à-dire apte à se fixer sur elle-même dans les zones où la bande de compression se recouvre, ce qui permet de fixer la bande de compression au fur et à mesure qu'elle est enroulée sur la partie du membre.

A cet effet, la bande de compression est par exemple enduite ou pulvérisée avec du latex, naturel ou synthétique, ce qui permet à une face de la bande d'adhérer sur la face opposée lorsque la bande est enroulée. Cependant, une telle bande de compression n'est pas entièrement satisfaisante car elle n'est pas lavable et réutilisable facilement. En effet, un passage en machine à laver tend à coller la bande sur elle-même et celle-ci sort de la machine avec l'aspect d'une boule de papier. Un fastidieux travail de décollage de la bande et de remise en forme est alors nécessaire pour pouvoir réappliquer la bande.

Une autre solution consiste à appliquer des bandes de fixation auto-agrippantes sur les faces de la bande de compression, de sorte que les faces de la bande peuvent se fixer de façon réversible l'une sur l'autre, à l'image d'une fixation de type Velcro^{®}. Cependant, l'ajout de telles bandes de fixation complexifie la réalisation de la bande de compression en nécessitant des étapes de réalisation supplémentaires après le tissage de la bande, la rend plus épaisse et les propriétés des bandes de fixation peuvent nuire à l'extensibilité de la bande de compression et la rendre ainsi moins efficace pour appliquer la pression souhaitée.

L'un des buts de l'invention est de pallier ces inconvénients en proposant une bande de compression auto-agrippante qui peut être réalisée facilement, dont l'enroulement et le maintien sur une partie de membre est simplifié et qui soit facilement lavable et réutilisable.

A cet effet, l'invention concerne une bande de compression thérapeutique s'étendant principalement selon une direction d'allongement et présentant une première face et une deuxième face, opposée à la première face, ladite bande comprenant un tissu formé de fils de trame s'étendant selon une direction transversale sensiblement perpendiculaire à la direction d'allongement et de fils de chaîne s'étendant sensiblement selon la direction d'allongement lesdits fils de chaîne comprenant au moins :
- des fils élastiques,
- des premiers fils s'étendant principalement sur au moins une partie de la première face et formant des premiers éléments de fixation sur ladite première face,
- des deuxièmes fils s'étendant principalement sur au moins une partie de la deuxième face et formant des deuxièmes éléments de fixation sur ladite deuxième face,
les deuxièmes éléments de fixation étant agencés pour coopérer avec les premiers éléments de fixation lorsqu'au moins une partie de la première face est appliquée sur au moins une partie de la deuxième face de sorte à fixer de façon réversible ladite première face sur ladite deuxième face.

Ainsi, la bande de compression selon l'invention intègre dans le tissu qui forme la bande les fils qui permettent de rendre la bande auto-agrippante et de fixer la bande sur elle-même au fur et à mesure qu'elle est appliquée sur la partie de membre à traiter. L'épaisseur de la bande de compression peut ainsi être maintenue à une valeur satisfaisante et les propriétés élastiques de la bande de compression sont conservées. La bande de compression selon l'invention est en outre lavable et réutilisable.

La bande de compression selon l'invention peut comprendre une ou plusieurs des caractéristiques suivantes, prises isolément ou selon toute combinaison techniquement envisageable :
- les premiers éléments de fixation sont formés par des premiers fils présentent chacun un premier diamètre de filament, les deuxièmes éléments de fixation présentant chacun une extrémité de fixation présentant une deuxième dimension supérieure au premier diamètre de filament, les extrémités de fixation des deuxièmes éléments de fixation étant agencées pour coopérer chacune avec au moins un premier élément de fixation pour fixer les deuxièmes éléments de fixation au premiers éléments de fixation lorsque la première face est appliquée sur la deuxième face ;
- les deuxièmes fils présentent un deuxième diamètre de filament supérieur ou égal au premier diamètre de filament ;
- les premiers éléments de fixation présentent une forme de boucle s'étendant en saillie d'au moins une partie de la première face ;
- les deuxièmes éléments de fixation présentent une forme d'élément d'ancrage s'étendant en saillie d'au moins une partie de la deuxième face, les éléments d'ancrage étant aptes à s'accrocher avec les boucles de la première face ;
- les fils élastiques sont des fils à base d'élastomère guipés et les premiers fils sont des fils en polyester ;
- les deuxièmes fils sont des fils en polypropylène présentant une température de fusion inférieure à la température de fusion du polyamide des fils élastiques et du polyester des premiers fils ;
- les fils de chaîne comprennent en outre des fils de protection s'étendant principalement entre les fils élastiques et la deuxième face ;
- la bande de compression présente une extensibilité sensiblement comprise entre 10% et 180% selon la direction d'allongement et une extensibilité sensiblement comprise entre 0% et 100% selon la direction transversale.

Selon un autre aspect, l'invention concerne un procédé de réalisation d'une bande de compression telle que décrite ci-dessus, comprenant les étapes suivantes :
- former un tissu comprenant des fils de trame et des fils de chaîne, lesdits fils de chaîne comprenant des fils élastiques, des premiers fils formant des flottés sur au moins une partie d'une première face du tissu et des deuxièmes fils formant des flottés sur au moins une partie d'une deuxième face du tissu, opposée à la première face,
- appliquer un traitement thermique ou mécanique aux flottés de deuxièmes fils de sorte à créer des deuxièmes éléments de fixation agencés pour coopérer avec les premiers éléments de fixation formés par les flottés de premiers fils lorsque la première face est appliquée sur la deuxième face de sorte à fixer de façon réversible ladite première face sur ladite deuxième face.

Une seule étape de traitement supplémentaire sur la bande pour réaliser les deuxièmes éléments de fixation est ainsi nécessaire pour obtenir une bande de compression auto-agrippante finie, à comparer à la réalisation normale d'une bande sèche de compression et de contention médicales. Le procédé de réalisation de la bande de compression reste donc simple.

D'autres aspects et avantages de l'invention apparaîtront à la lecture de la description qui suit, donnée à titre d'exemple et faite en référence aux dessins annexés, dans lesquels :
[Fig 1] - la Fig. 1 est une représentation schématique en coupe d'une bande de compression selon l'invention,
[Fig 2] - la Fig. 2 est une représentation schématique en coupe du tissu formant une bande de compression selon un mode de réalisation de l'invention,
[Fig 3] - la Fig. 3 est une représentation schématique de l'armure de la bande de compression de la Fig. 2,
[Fig 4] - la Fig. 4 est une représentation schématique en perspective d'une zone agrandie de la bande de compression dans laquelle un deuxième élément de fixation coopère avec des premiers éléments de fixation, et
[Fig 5] - la Fig. 5 est une représentation schématique en perspective d'une partie d'un membre d'un patient sur laquelle une bande de compression est appliquée.

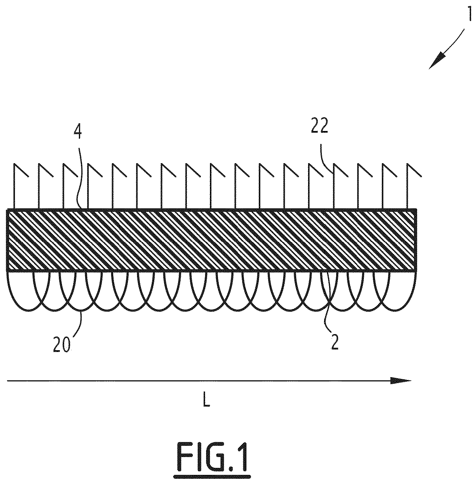

En référence à la Fig. 1, on décrit une bande de compression thérapeutique 1 s'étendant principalement selon une direction d'allongement L et comprenant une première face 2 et une deuxième face 4, opposée à la première face 2. La longueur de la bande de compression 1 correspond à la dimension de la bande de compression selon la direction d'allongement L et la largeur de la bande correspond à la dimension de la bande selon une direction perpendiculaire à la direction d'allongement L, dite direction transversale T.

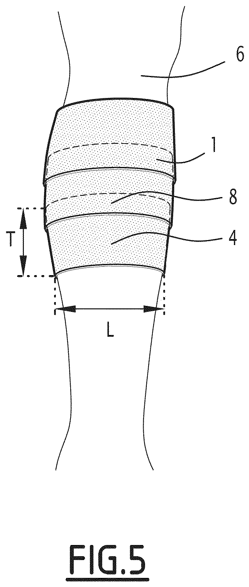

La première face 2 est appliquée sur la peau d'un patient lorsque la bande de compression 1 est enroulée autour d'au moins une partie d'un membre 6 du patient, comme représenté sur la Fig. 5, et la deuxième face est tournée vers l'extérieur. Par convention, les termes « inférieur », « supérieur », « en-dessous », « au-dessus », etc. sont définis par rapport à la direction allant de la première face 2 à la deuxième face 4, correspondant à l'épaisseur de la bande de compression 1. Ainsi, la première face 2 forme la face inférieure de la bande de compression 1 et la deuxième face 4 forme la face supérieure de la bande de compression 1.

Comme représenté sur la Fig. 5, lorsque la bande de compression 1 est enroulée sur la partie de membre 6, la bande de compression 1 se recouvre partiellement, c'est-à-dire qu'une partie de la première face 2 d'une spire supérieure est appliquée sur une partie de la deuxième face 4 d'une spire inférieure dans une zone de recouvrement 8, une spire correspondant à un tour de bande autour de la partie de membre 6. La zone de recouvrement 8 s'étend par exemple sur sensiblement la moitié de la largeur de la bande de compression. En variante, le recouvrement peut se faire sur moins de la moitié de la largeur de la bande ou sur plus de la moitié, par exemple sur les deux tiers de la largeur de la bande.

La bande de compression 1 est extensible au moins selon la direction d'allongement L. En d'autres termes, la bande de compression 1 peut être étirée selon la direction d'allongement L et retrouver sa longueur initiale lorsque l'étirement cesse d'être appliqué sur la bande de compression. La bande de compression 1 présente par exemple un allongement compris entre 10% et 180% selon la direction d'allongement. Comme connu en soit, l'étirement de la bande de compression selon la direction d'allongement L permet d'ajuster la pression exercée par la bande de compression sur la partie de membre 6 autour de laquelle la bande de compression est enroulée. Selon un mode de réalisation, la bande de compression 1 est en outre extensible selon la direction transversale T. On parle alors de bande de compression bi-extensible. L'extensibilité selon la direction transversale T est par exemple comprise entre 0% et 100%, 0% correspondant une bande de compression 1 extensible uniquement selon la direction d'allongement L. Pour l'extensibilité selon la direction d'allongement et selon la direction transversale, l'allongement est mesuré suivant les spécifications de la norme NF EN ISO 20932 du 1^{er} Février 2020. Comme connu en soit, l'extensibilité selon la direction transversale T permet de faciliter la pose de la bande de compression, car celle-ci s'adapte naturellement à la morphologie de la partie de membre 6 autour de laquelle la bande est enroulée, et de réduire le risque de formation de plis dans la bande au cours de la pose.

La bande de compression 1 est formée par un tissu comprenant des fils de trame 10 et des fils de chaînes 12.

Les fils de trame 10 s'étendent selon la direction transversale T. Les fils de trame 10 sont par exemple des fils de polyester. En variante, les fils de trame peuvent être tout fil textile en matière d'origine naturelle, synthétique ou artificielle. En effet, le matériau des fils de trame n'influe pas sur les propriétés de compression et de fixation de la bande de compression, ces propriétés étant conférées à la bande par les fils de chaînes, comme cela sera décrit ultérieurement. Selon un mode de réalisation, les fils de trame sont ou comprennent des fils élastiques, tels que des fils en matériau synthétique texturé, des filés de fibres à torsion élevée ou des fils à base d'élastomère, afin de former un tissu extensible selon la direction transversale T. En variante, l'extensibilité selon la direction transversale T est obtenue par le tissage de fils de chaine élastiques en pas de gaze.

Les fils de chaînes 12 comprennent au moins des fils élastiques 14, des premiers fils 16, s'étendant principalement sur au moins une partie de la première face 2, et des deuxième fils 18 s'étendant principalement sur au moins une partie de la deuxième face 4.

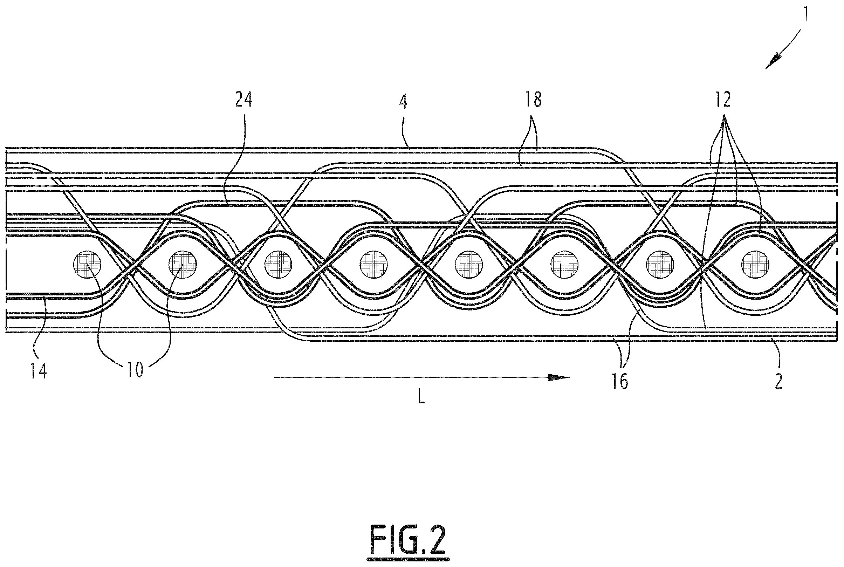

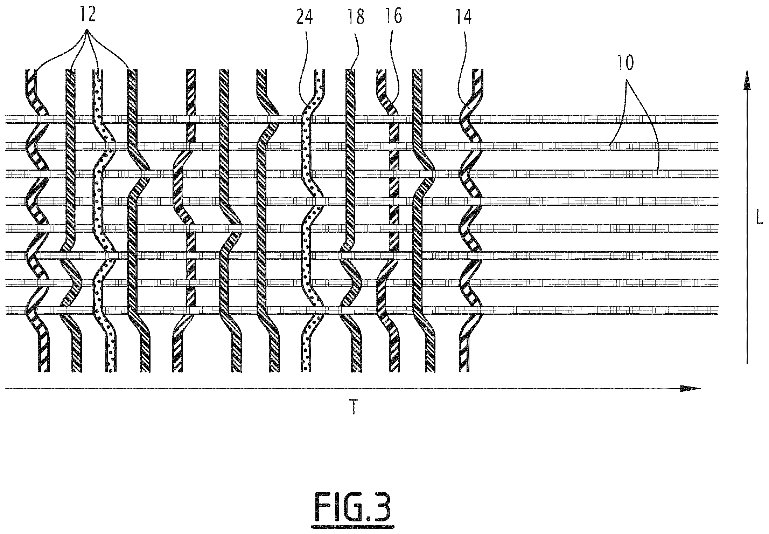

Les fils élastiques 14 sont agencés pour conférer à la bande de compression son extensibilité selon la direction longitudinale L. Les fils élastiques 14 sont par exemple des fils en matériau synthétique texturé, des filés de fibres à torsion élevée ou des fils à base d'élastomère guipés. Selon un mode de réalisation, les fils élastiques sont des fils d'élasthanne guipés par du polyester. Comme représenté sur le Figs 2 et 3, les fils élastiques 14 passent successivement au-dessus et en-dessous des fils de trame 10 selon la direction d'allongement L. Une alternance entre des fils élastiques 14 adjacents selon la direction transversale T peut être prévue afin que, lorsqu'un fil élastique 14 passe au-dessus d'un fil de trame 10, un fil élastique 14 adjacent passe en-dessous de ce fil de trame 10. Les fils élastiques 14 s'étendent sur toute la longueur et sont répartis sur toute la largeur du tissu formant la bande de compression 1. Les fils élastiques 14 s'étendent entre la première face 2 et la deuxième face 4 et forment avec les fils de trame 10 les fils structurant du tissu formant la bande de compression 1.

Comme indiqué précédemment, les premiers fils 16 s'étendent principalement sur au moins une partie de la première face 2. Par « s'étendant principalement sur au moins une partie de la première face 2 », on entend que les premiers fils s'étendent sur la plus grande partie de leur longueur du côté de la première face 2 et qu'au moins une partie de la première face 2 est formée en surface de celle-ci par des premiers fils 16. Pour ce faire, les premiers fils 16 passent successivement en dessous d'un certain nombre de fils de trame 10, ce nombre étant supérieur au nombre de fils de trame 10 au-dessus desquels passent les premiers fils. Ainsi, comme représenté à titre d'exemple sur les Figs. 2 et 3, les premiers fils 16 passent en dessous de plus de deux fils de trame 10 puis au-dessus d'un ou deux fils de trame 10 puis, à nouveau en dessous de plus de deux fils de trame 10 selon la direction d'allongement L. On comprend ainsi que les premiers fils 16 s'étendent plus du côté de la première face 2 que du côté de la deuxième face 4. Selon un exemple particulier, comme représenté sur la Fig. 3, une partie des premiers fils 16 passent en dessous de six fils de trame 10 successifs puis au-dessus de deux fils de trame 10 puis à nouveau en dessous de six fils de trame 10. Une autre partie des premiers fils 16 passent en dessous de quatre fils de trame 10 puis au-dessus de deux fils de trame 10 puis à nouveau en dessous de deux fils de trame 10. En variante, un premier fil 16 peut passer en alternance en dessous d'un nombre variable de fils de trame 10, par exemple six puis trois, et au-dessus d'un ou deux fils de trame 10. Ainsi, les premiers fils 16 forment des flottés sur la première face 2 de la bande de compression 1. Une alternance entre des premiers fils 16 adjacents selon la direction transversale T peut être prévue afin que les premiers fils 16 ne passent pas toujours au-dessus des mêmes fils de trame 10, comme visible sur les Figs 2 et 3.

Les premiers fils 16 s'étendent sur toute la longueur du tissu formant la bande de compression 1. Selon la direction transversale T, les premiers fils 16 s'étendent sur la première face 2 au moins dans la zone de recouvrement 8 de la bande de compression 1. En d'autres termes, les premiers fils 16 ne peuvent être répartis que sur une partie de la largeur du tissu formant la bande de compression 1. En effet, comme cela sera décrit ultérieurement, les premiers fils 16 servent à assurer la fixation de la bande de compression sur elle-même et ne peuvent donc être utilisés que dans la zone de recouvrement 8 de la bande de compression.

Les premiers fils 16 sont par exemple des fils en polyester composés de filaments présentant un premier diamètre. Le premier diamètre de filament est par exemple compris entre 0.001 mm et 0.03 mm. Selon un mode de réalisation particulier, les premiers fils 16 sont des fils de polyester PES1/330/576, signifiant que chaque fil possède 576 filaments pour un titre total de 330dtex, soit 0,57dtex par filament. De tels fils procurent une certaine douceur sur la peau du patient, la première face 2 étant appliquée sur la peau comme décrit précédemment.

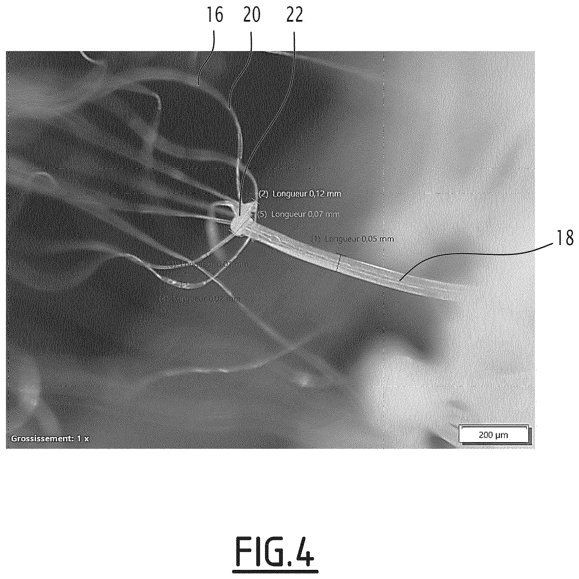

Les flottés de premiers fils 16 sur la deuxième face 2 du tissu permettent aux premiers fils 16 de former des premiers éléments de fixation 20 sur la première face 2, comme cela sera décrit plus en détail ultérieurement. Plus particulièrement, les premiers éléments de fixation 20 se présentent sous la forme de boucles ou bouclettes, s'étendant en saillie d'au moins une partie de la première face 2, comme représenté schématiquement sur la Fig. 1 et comme visible plus en détail sur la Fig. 4.

Comme indiqué précédemment, les deuxièmes fils 18 s'étendent principalement sur au moins une partie de la deuxième face 4. Par « s'étendant principalement sur au moins une partie de la deuxième face 4 », on entend que les deuxièmes fils s'étendent sur la plus grande partie de leur longueur du côté de la deuxième face 4 et qu'au moins une partie de la deuxième face 4 est formée en surface de celle-ci par des deuxièmes fils 18. Pour ce faire, les deuxièmes fils 18 passent successivement au-dessus d'un certain nombre de fils de trame 10, ce nombre étant supérieur au nombre de fils de trame 10 en dessous desquels passent les deuxièmes fils. Ainsi, comme représenté à titre d'exemple sur les Figs. 2 et 3, les deuxièmes fils 18 passent au-dessus de plus d'un fil de trame 10 puis en dessous d'un fil de trame 10 puis, à nouveau au-dessus de plus d'un fil de trame 10 selon la direction d'allongement L. On comprend ainsi que les deuxièmes fils 18 s'étendent plus du côté de la deuxième face 4 que du côté de la première face 2. Selon un exemple particulier, comme représenté sur la Fig. 3, une partie des deuxièmes fils 18 passent au-dessus de sept fils de trame 10 successifs puis en dessous d'un fil de trame 10 puis à nouveau au-dessus de sept fils de trame 10. En variante, un deuxième fil 18 peut passer en alternance au-dessus d'un nombre variable de fils de trame 10, par exemple sept puis cinq, et en dessous d'un ou deux fils de trame 10. Ainsi, les deuxièmes fils 18 forment des flottés sur la deuxième face 4 de la bande de compression 1 avant d'être traités, comme cela sera décrit ultérieurement. Une alternance entre des deuxièmes fils 18 adjacents selon la direction transversale T peut être prévue afin que les deuxièmes fils 18 ne passent pas toujours en dessous des mêmes fils de trame 10, comme visible sur les Figs 2 et 3.

Les deuxièmes fils 18 s'étendant sur toute la longueur du tissu formant la bande de compression 1. Selon la direction transversale T, les deuxièmes fils 18 s'étendent sur la deuxième face 4 au moins dans la zone de recouvrement 8 de la bande de compression 1. En d'autres termes, les deuxièmes fils 18 ne sont pas nécessairement répartis sur toute la largeur du tissu formant la bande de compression 1. En effet, comme cela sera décrit ultérieurement, les deuxièmes fils 18 servent à assurer la fixation de la bande de compression sur elle-même en coopération avec les premiers fils 16 et ne peuvent donc être utilisés que dans la zone de recouvrement 8 de la bande de compression.

Selon un mode de réalisation, les deuxièmes fils 18 sont des fils en polyamide présentant un deuxième diamètre de filament. Le deuxième diamètre est supérieur ou égal au premier diamètre. Ainsi, le deuxième diamètre est par exemple compris entre 0.01 mm et 0.07 mm, par exemple sensiblement égal à 0.05 mm. Selon un mode de réalisation particulier, les deuxièmes fils 18 sont des fils de polyamide PA 2/110/5 signifiant que chaque fil possède 5 filaments pour un titre total de 110dtex, soit 22dtex par filament.

Comme cela sera décrit plus en détail ultérieurement, les flottés de deuxièmes fils 18 sur la deuxième face 4 sont soumis à un traitement mécanique ou thermique pour former des deuxièmes éléments de fixation 22 agencés pour coopérer avec les premiers éléments de fixation 20 s'étendant sur la première face 2.

Lorsque les deuxièmes fils 18 sont réalisés en polyamide, ils présentent une température de fusion proche de celle des fils élastiques 14 et un traitement thermique des deuxièmes fils 18 risque donc de dégrader en même temps les fils élastiques 14. Pour éviter cette dégradation, le tissu formant la bande de compression comprend en outre selon un mode de réalisation des fils de protection 24 s'étendant principalement entre les fils élastiques 14 et la deuxième face 4. Ces fils de protection 24 sont agencés pour protéger les fils élastiques 14, ainsi que les fils de trame 10 et les deuxièmes fils 16, du traitement thermique appliqué aux deuxièmes fils 18. A cet effet, les fils de protection 24 sont interposés entre la partie des deuxièmes fils 18 qui s'étendent sur la deuxième face 4 et le reste du tissu de sorte qu'ils seront soumis au traitement des deuxièmes fils en lieu et place des fils élastiques 14 et des fils de trame 10. Les fils de protection 24 sont par exemple des fils de polyamide ou de polyester texturé. Les fils de protection 24 sont intégrés dans le tissu, par exemple en passant au-dessus de trois fils de trame 10 puis en dessous d'un fil de trame 10 puis à nouveau au-dessus de trois fils de trame 10, comme représenté sur les Figs. 2 et 3.

L'ajout de fils de protection 24 augmente nécessairement l'épaisseur de la bande de compression 1, ce qui peut être désavantageux dans certains cas. Afin d'éviter la présence de tels fils de protection 24, les deuxièmes fils 18 sont réalisés selon un autre mode de réalisation en un matériau présentant une température de fusion inférieure à celle des fils s'étendant en dessous des deuxièmes fils 18, c'est-à-dire au moins les fils de trame 10 et les fils élastiques 14, voire les premiers fils 16. A cet effet, les deuxièmes fils 18 sont par exemple des fils en polypropylène qui présente une température de fusion voisine de 160°C alors que cette température est supérieure à 200°C pour les matériaux, tels que le polyamide ou le polyester, utilisés pour réaliser les fils de trame 10 et les fils élastiques 16. Selon ce mode de réalisation, le tissu formant la bande de compression 1 ne comprend pas de fils de protection et présente ainsi une épaisseur réduite par rapport au mode de réalisation dans lequel des fils de protection sont prévus. A titre d'exemple, la bande de compression présente une épaisseur comprise entre 1 mm et 3mm, mesurée suivant la norme NF EN ISO 5084:2016.

Comme indiqué précédemment, le traitement mécanique ou thermique des deuxièmes fils 18 est agencé pour former des deuxièmes éléments de fixation 22 sur au moins une partie de la deuxième face 4 s'étendant en regard de la partie de la première face 2 qui comprend les premiers éléments de fixation 20. Le traitement mécanique ou thermique vise à couper, à raser ou à fondre les flottés de deuxièmes fils 18 pour que les extrémités créées des deuxièmes fils 18 forment des extrémités de fixation des deuxièmes éléments de fixation 22, telles que des éléments d'ancrage, capables de s'accrocher ou de s'ancrer avec ou dans au moins un premier élément de fixation 20, comme représenté de façon schématique sur la Fig. 1 et comme visible plus en détail sur la Fig. 4. Pour ce faire, les extrémités de fixation des deuxièmes fils 18 présentent une dimension, telle qu'une largeur ou un diamètre, supérieure au premier diamètre des filaments des premiers fils 16. A titre d'exemple, la dimension des extrémités de fixation est comprise entre 0.04 mm et 0.12 mm. Une telle dimension peut être obtenue du fait du deuxième diamètre des deuxièmes fils 18 et/ou par fusion d'extrémités de fixation entre elles au cours du traitement thermique des deuxièmes fils 18. Le traitement thermique des deuxièmes fils 18 est par exemple un traitement au laser ou un traitement au plasma obtenu en exposant la deuxième face 4 à un rayonnement laser ou un rayonnement plasma agencé pour couper ou fondre les deuxièmes fils 18 afin de former les deuxièmes éléments de fixation 22. Le traitement thermique est tel que les extrémités de fixation peuvent former des crochets du type de ceux prévus dans une fixation Velcro^{®}, comme représenté de façon schématique sur la Fig. 1 ou les extrémités de fixation présentent une forme de tête de champignon comme représenté sur la Fig. 4. Plus généralement, les deuxièmes éléments de fixation 22 présentent chacun une forme d'élément d'ancrage s'étendant en saillie d'au moins une partie de la deuxième face 4. De tels deuxièmes éléments de fixation 22 peuvent chacun coopérer avec un ou plusieurs premiers éléments de fixation 20 par entremêlement.

En variante, les deuxièmes fils 18 sont soumis à un traitement mécanique, qui consiste par exemple en un rasage par tonte des flottés des deuxièmes fils 18. Une telle tonte permet également d'obtenir des deuxièmes éléments de fixation 22 sur au moins une partie de la deuxième face 4.

La coopération des premiers éléments de fixation 20 et les deuxièmes éléments de fixation 22 est telle que la première face 2 est fixée de façon réversible à la deuxième face 4 lorsque ces faces sont appliquées l'une contre l'autre dans la zone de recouvrement. Plus particulièrement, la force auto-agrippante de la bande de compression 1 lorsqu'elle est fixée sur elle-même par la coopération des premiers éléments de fixation 20 et des deuxièmes éléments de fixation 22 est supérieure ou égale à 5 cN/cm, mesurée suivant le norme NF S 97-115.

La bande de compression a été décrite avec des premiers éléments de fixation formés par des boucles sur la première face 2 et des deuxièmes éléments de fixation formés par des éléments d'ancrage aptes à coopérer avec ces boucles sur la deuxième face 4. Il est cependant entendu que les boucles pourraient s'étendre sur la deuxième face 4 et que les éléments d'ancrage pourraient s'étendre sur la première face 2, bien que cela réduise le confort de la bande de compression car les boucles ont un contact plus doux avec la peau. Il est entendu que les premiers éléments de fixation 20 et les deuxièmes éléments de fixation 22 peuvent s'étendre respectivement sur toute la première face 2 et sur toute la deuxième face 4 selon la direction transversale T.

La pose de la bande de compression est simple car la bande de compression 1 peut être appliquée à l'état étiré tout en se fixant sur elle-même dans la zone de recouvrement 8 au fur et à mesure qu'elle est enroulée autour de la partie de membre 6. En effet, dans la ou les zones de recouvrement 8, la première face 2 est appliquée sur la deuxième face 4 ce qui entraîne la mise en contact des premiers éléments de fixation 20 avec les deuxièmes éléments de fixation 22 et leur coopération, comme représenté sur la Fig. 4, pour fixer la bande de compression 1 sur elle-même. On assure ainsi une fixation robuste et durable de la bande de compression sur elle-même lorsqu'elle est enroulée autour de la partie de membre 6. Plus particulièrement la bande de compression 1 est maintenue fermement autour de la partie de membre 6 en exerçant la pression souhaitée sur celle-ci et n'a pas tendance à glisser le long du membre le temps que la bande de compression 1 est portée par un patient car la bande de compression est fixée sur elle-même sur toute sa longueur et pas seulement à une extrémité de celle-ci, comme c'est souvent le cas. En outre, la pose de la bande de compression 1 ne nécessite pas l'utilisation d'élément de fixation supplémentaire, tel qu'une agrafe ou autre. De plus, la bande de compression est lavable et réutilisable, la bande de compression n'ayant pas tendance à se fixer sur elle-même en forme de boule de papier lorsqu'elle passe à la machine à laver.

Le procédé de réalisation de la bande de compression 1 est également relativement simple puisque tous les fils, à savoir les fils de trame 10, les fils élastiques 14, les premiers fils 16, les deuxièmes fils 18 et, éventuellement, les fils de protection 24 sont tissés ensemble pour former le tissu formant la bande de compression 1. Le tissage est tel que les premiers fils 16 forment des flottés sur au moins une partie de la première face 2 et les deuxième fils 18 forment des flottés sur au moins une partie de la deuxième face 4. Seul le traitement permettant d'obtenir les deuxièmes éléments de fixation 22 à partir des flottés de deuxièmes fils 18 sur la deuxième face 4 s'ajoute aux étapes de fabrication normale d'une bande de compression pour obtenir la bande de compression 1 finie. La réalisation de la bande de compression 1 peut donc se faire en peu d'étapes.

## Revendications

1. Bande de compression (1) thérapeutique s'étendant principalement selon une direction d'allongement (L) et présentant une première face (2) et une deuxième face (4), opposée à la première face (2), ladite bande comprenant un tissu formé de fils de trame (10) s'étendant selon une direction transversale (T) sensiblement perpendiculaire à la direction d'allongement (L), et de fils de chaîne (12) s'étendant sensiblement selon la direction d'allongement (L), lesdits fils de chaîne (12) comprenant au moins :
- des fils élastiques (14),
- des premiers fils (16) s'étendant principalement sur au moins une partie de la première face (2) et formant des premiers éléments de fixation (20) sur ladite première face (2),
- des deuxièmes fils (18) s'étendant principalement sur au moins une partie de la deuxième face (4) et formant des deuxièmes éléments de fixation (22) sur ladite deuxième face (4),
les deuxièmes éléments de fixation (22) étant agencés pour coopérer avec les premiers éléments de fixation (20) lorsqu'au moins une partie de la première face (2) est appliquée sur au moins une partie de la deuxième face (4) de sorte à fixer de façon réversible ladite première face (2) sur ladite deuxième face (4).

2. Bande de compression selon la revendication 1, dans laquelle les premiers éléments de fixation (20) sont formés par des premiers fils (16) présentent chacun un premier diamètre de filament, les deuxièmes éléments de fixation (22) présentant chacun une extrémité de fixation présentant une deuxième dimension supérieure au premier diamètre de filament, les extrémités de fixation des deuxièmes éléments de fixation (22) étant agencées pour coopérer chacune avec au moins un premier élément de fixation (20) pour fixer les deuxièmes éléments de fixation (22) au premiers éléments de fixation (20) lorsque la première face (2) est appliquée sur la deuxième face (4).

3. Bande de compression selon la revendication 2, dans laquelle les deuxièmes fils (18) présentent un deuxième diamètre de filament supérieur ou égal au premier diamètre de filament.

4. Bande de compression selon l'une quelconque des revendications 1 à 3, dans laquelle les premiers éléments de fixation (20) présentent une forme de boucle s'étendant en saillie d'au moins une partie de la première face (2).

5. Bande de compression selon la revendication 4, dans lequel les deuxièmes éléments de fixation (22) présentent une forme d'élément d'ancrage s'étendant en saillie d'au moins une partie de la deuxième face (4), les éléments d'ancrage étant aptes à s'accrocher avec les boucles de la première face (2).

6. Bande de compression selon l'une quelconque des revendications 1 à 5, dans laquelle les fils élastiques (14) sont des fils à base d'élastomère guipés et les premiers fils (16) sont des fils en polyester.

7. Bande de compression selon la revendication 6, dans laquelle les deuxièmes fils (18) sont des fils en polypropylène présentant une température de fusion inférieure à la température de fusion des fils élastiques (14) et du polyester des premiers fils (16).

8. Bande de compression selon l'une quelconque des revendications 1 à 7, dans laquelle les fils de chaîne (12) comprennent en outre des fils de protection (24) s'étendant principalement entre les fils élastiques (14) et la deuxième face (4).

9. Bande de compression selon l'une quelconque des revendications 1 à 8, présentant une extensibilité sensiblement comprise entre 10% et 180% selon la direction d'allongement (L) et une extensibilité sensiblement comprise entre 0% et 100% selon la direction transversale (T).

10. Procédé de réalisation d'une bande de compression selon l'une quelconque des revendications 1 à 9, comprenant les étapes suivantes :
- former un tissu comprenant des fils de trame (10) et des fils de chaîne (12), lesdits fils de chaîne (12) comprenant des fils élastiques (14), des premiers fils (16) formant des flottés sur au moins une partie d'une première face (2) du tissu et des deuxièmes fils (18) formant des flottés sur au moins une partie d'une deuxième face (4) du tissu, opposée à la première face (2),
- appliquer un traitement thermique ou mécanique aux flottés de deuxièmes fils (18) de sorte à créer des deuxièmes éléments de fixation (22) agencés pour coopérer avec les premiers éléments de fixation (20) formés par les flottés de premiers fils (16) lorsque la première face (2) est appliquée sur la deuxième face (4) de sorte à fixer de façon réversible ladite première face (2) sur ladite deuxième face (4).

## Patentansprüche

1. Therapeutische Kompressionsbinde (1), die sich hauptsächlich in einer Dehnungsrichtung (L) erstreckt und eine erste Seite (2) und eine zweite Seite (4) gegenüber der ersten Seite (2) aufweist, die Binde umfassend ein Gewebe, das aus Schussfäden (10) gebildet ist, die sich in einer Querrichtung (T) im Wesentlichen senkrecht zu der Dehnungsrichtung (L) erstrecken, und Kettfäden (12), die sich im Wesentlichen in der Dehnungsrichtung (L) erstrecken, die Kettfäden (12) mindestens umfassend:
- elastische Fäden (14),
- erste Fäden (16), die sich hauptsächlich über mindestens einen Teil der ersten Seite (2) erstrecken und auf der ersten Seite (2) erste Befestigungselemente (20) bilden,
- zweite Fäden (18), die sich hauptsächlich über mindestens einen Teil der zweiten Seite (4) erstrecken und auf der zweiten Seite (4) zweite Befestigungselemente (22) bilden, wobei die zweiten Befestigungselemente (22) angeordnet sind, um mit den ersten Befestigungselementen (20) zusammenzuwirken, wenn mindestens ein Teil der ersten Seite (2) auf mindestens einen Teil der zweiten Seite (4) aufgebracht ist, um die erste Seite (2) reversibel auf der zweiten Seite (4) zu befestigen.

2. Kompressionsbinde nach Anspruch 1, wobei die ersten Befestigungselemente (20) durch erste Fäden (16) gebildet sind, die jeweils einen ersten Filamentdurchmesser aufweisen, die zweiten Befestigungselemente (22) jeweils ein Befestigungsende aufweisen, das eine zweite Abmessung größer als der erste Filamentdurchmesser aufweist, wobei die Befestigungsenden der zweiten Befestigungselemente (22) angeordnet sind, um jeweils mit mindestens einem ersten Befestigungselement (20) zusammenzuwirken, um die zweiten Befestigungselemente (22) an den ersten Befestigungselementen (20) zu befestigen, wenn die erste Seite (2) auf die zweite Seite (4) aufgebracht ist.

3. Kompressionsbinde nach Anspruch 2, wobei die zweiten Fäden (18) einen zweiten Filamentdurchmesser größer als oder gleich dem ersten Filamentdurchmesser aufweisen.

4. Kompressionsbinde nach einem der Ansprüche 1 bis 3,
wobei die ersten Befestigungselemente (20) eine Schlaufenform aufweisen, die sich hervorstehend von mindestens einem Teil der ersten Seite (2) erstreckt.

5. Kompressionsbinde nach Anspruch 4, wobei die zweiten Befestigungselemente (22) eine Verankerungselementform aufweisen, die sich hervorstehend von mindestens einem Teil der zweiten Seite (4) erstreckt, wobei die Verankerungselemente geeignet sind, um sich mit den Schlaufen der ersten Seite (2) zu verhaken.

6. Kompressionsbinde nach einem der Ansprüche 1 bis 5, wobei die elastischen Fäden (14) umsponnene Fäden auf Basis von Elastomer sind und die ersten Fäden (16) Fäden aus Polyester sind.

7. Kompressionsbinde nach Anspruch 6, wobei die zweiten Fäden (18) Fäden aus Polypropylen sind, die eine niedrigere Schmelztemperatur aufweisen als die Schmelztemperatur der elastischen Fäden (14) und des Polyesters der ersten Fäden (16).

8. Kompressionsbinde nach einem der Ansprüche 1 bis 7, wobei die Kettfäden (12) ferner Schutzfäden (24) umfassen, die sich hauptsächlich zwischen den elastischen Fäden (14) und der zweiten Seite (4) erstrecken.

9. Kompressionsbinde nach einem der Ansprüche 1 bis 8, die eine Streckbarkeit, die im Wesentlichen zwischen 10 % und 180 % in der Dehnungsrichtung (L) liegt, und eine Streckbarkeit, die im Wesentlichen zwischen 0 % und 100 % in der Querrichtung (T) liegt, aufweist.

10. Verfahren zur Herstellung einer Kompressionsbinde nach einem der Ansprüche 1 bis 9, umfassend die folgenden Schritte:
- Bilden eines Gewebes, umfassend Schussfäden (10) und Kettfäden (12), die Kettfäden (12) umfassend elastische Fäden (14), erste Fäden (16), die Flottierungen auf mindestens einem Teil einer ersten Seite (2) des Gewebes bilden, und zweite Fäden (18), die Flottierungen auf mindestens einem Teil einer zweiten Seite (4) des Gewebes gegenüber der ersten Seite (2) bilden,
- Anwenden einer Wärmebehandlung oder mechanischen Behandlung auf die Flottierungen von zweiten Fäden (18), um zweite Befestigungselemente (22) zu erzeugen, die angeordnet sind, um mit den ersten Befestigungselementen (20) zusammenzuwirken, die durch die Flottierungen von ersten Fäden (16) gebildet werden, wenn die erste Seite (2) auf die zweite Seite (4) aufgebracht wird, um die erste Seite (2) reversibel auf der zweiten Seite (4) zu befestigen.

## Claims

1. A therapeutic compression strip (1) extending mainly in a direction of elongation (L) and having a first face (2) and a second face (4), opposite the first face (2), said strip comprising a fabric formed of weft yarns (10) extending in a transverse direction (T) substantially perpendicular to the direction of elongation (L), and of warp yarns (12) extending substantially in the direction of elongation (L), said warp yarns (12) comprising at least:
- elastic yarns (14),
- first yarns (16) extending mainly over at least part of the first face (2) and forming first fastening elements (20) on said first face (2),
- second yarns (18) extending mainly over at least part of the second face (4) and forming second fastening elements (22) on said second face (4),
the second fastening elements (22) being arranged to cooperate with the first fastening elements (20) when at least part of the first face (2) is applied to at least part of the second face (4) so as to reversibly fasten said first face (2) to said second face (4).

2. The compression strip according to claim 1, wherein the first fastening elements (20) are formed by first yarns (16) each having a first filament diameter, the second fastening elements (22) each having a fastening end having a second dimension greater than the first filament diameter, the fastening ends of the second fastening elements (22) being arranged to each cooperate with at least one first fastening element (20) for fastening the second fastening elements (22) to the first fastening elements (20) when the first face (2) is applied to the second face (4).

3. The compression strip according to claim 2, wherein the second yarns (18) have a second filament diameter greater than or equal to the first filament diameter.

4. The compression strip according to any one of claims 1 to 3, wherein the first fastening elements (20) have the shape of a loop projecting from at least part of the first face (2).

5. The compression strip according to claim 4, wherein the second fastening elements (22) have the form of an anchoring element projecting from at least part of the second face (4), the anchoring elements being able to hook with the loops of the first face (2).

6. The compression strip according to any one of claims 1 to 5, wherein the elastic yarns (14) are covered elastomer-based yarns and the first yarns (16) are polyester yarns.

7. The compression strip according to claim 6, wherein the second yarns (18) are polypropylene yarns having a melting temperature lower than the melting temperature of the elastic yarns (14) and of the polyester of the first yarns (16).

8. The compression strip according to any one of claims 1 to 7, wherein the warp yarns (12) further comprise protective yarns (24) extending mainly between the elastic yarns (14) and the second face (4).

9. The compression strip according to any one of claims 1 to 8, having an extensibility substantially between 10% and 180% in the direction of elongation (L) and an extensibility substantially between 0% and 100% in the transverse direction (T).

10. A method for producing a compression strip according to any one of claims 1 to 9, comprising the following steps:
- forming a fabric comprising weft yarns (10) and warp yarns (12), said warp yarns (12) comprising elastic yarns (14), first yarns (16) forming floats over at least part of a first face (2) of the fabric and second yarns (18) forming floats over at least part of a second face (4) of the fabric, opposite the first face (2),
- applying a thermal or mechanical treatment to the floats of second yarns (18) so as to create second fastening elements (22) arranged to cooperate with the first fastening elements (20) formed by the floats of first yarns (16) when the first face (2) is applied to the second face (4) so as to reversibly fasten said first face (2) to said second face (4).
